# EUROPEAN PATENT SPECIFICATION

(11) **EP 3 795 995 B1**
(45) Date of publication and mention of the grant of the patent: **27.12.2023**
(21) Application number: 20205764.2
(22) Date of filing: 03.03.2016
(51) Int. Cl.: G01N 33/12, H01J 49/00

(54) **SIMULTANEOUSLY DETECTION OF OFF-NOTE OR BOAR TAINT RELATED COMPOUNDS IN ANIMAL TISSUE**
GLEICHZEITIGE DETEKTION VON VERBINDUNGEN IM ZUSAMMENHANG MIT NACHGESCHMACK ODER EBERGERUCH IN TIERGEWEBE
DÉTECTION SIMULTANÉE DE COMPOSÉS LIÉS À UNE ODEUR ATYPIQUE OU SEXUELLE DANS DES TISSUS ANIMAUX

(30) Priority: 03.03.2015 DK PA201500120
(43) Date of publication of application: 24.03.2021
(62) Divisional of application: 16707471.5
(73) Proprietor: Teknologisk Institut, 2630 Taastrup (DK)
(72) Inventor: LUND, Birgitte Lange Winther, 2630 Taastrup (DK); MEINERT, Lene, 2630 Taastrup (DK); BORGGARD, Claus, 2630 Taastrup (DK)
(74) Representative: AWA Denmark A/S

(56) References cited:
- EP-A1- 1 233 267
- SØRENSEN K M ET AL: "Measurement of boar taint in porcine fat using a high-throughput gas chromatography-mass spectrometry protocol", JOURNAL OF AGRICULTURAL AND FOOD CHEMISTRY, vol. 62, no. 39, 17 September 2014 (2014-09-17), pages 9420-9427, XP055223069, ISSN: 0021-8561, DOI: 10.1021/jf5022785
- LOHNE J J ET AL: "Laser diode thermal desorption mass spectrometry for the analysis of quinolone antibiotic residues in aquacultured seafood", RAPID COMMUNICATIONS IN MASS SPECTROMETRY., vol. 26, no. 24, 5 November 2012 (2012-11-05), pages 2854-2864, XP055272575, GB ISSN: 0951-4198, DOI: 10.1002/rcm.6414
- LANSHOEFT C ET AL: "Laser diode thermal desorption atmospheric pressure chemical ionization tandem mass spectrometry applied for the ultra-fast quantitative analysis of BKM120 in human plasma", ANALYTICAL AND BIOANALYTICAL CHEMISTRY, vol. 406, no. 22, 24 June 2014 (2014-06-24), pages 5413-5423, XP055272586, DE ISSN: 1618-2642, DOI: 10.1007/s00216-014-7966-6
- FISCHER J ET AL: "Determination of the boar taint compound skatole in meat juice by means of stable isotope dilution analysis direct immersion solid phase microextraction gas chromatography/mass spectrometry", MEAT SCIENCE, ELSEVIER SCIENCE, GB, vol. 91, no. 3, 26 January 2012 (2012-01-26), pages 261-265, XP028406850, ISSN: 0309-1740, DOI: 10.1016/J.MEATSCI.2012.01.024 [retrieved on 2012-02-11]
- Buttinger G ET AL: "In house validation of a reference method for the determination of boar taint compounds by LC-MSMS" In: "European Commision Reports", 2014, XP055223079, ISSN: 1831-9424 ISBN: 978-92-7-935417-5 pages 1-45, * page 5, paragraph 6 - paragraph 9 * * page 31, paragraph 2 - page 34, paragraph 2 *
- FISCHER J ET AL: "Fast and solvent-free quantitation of boar taint odorants in pig fat by stable isotope dilution analysis-dynamic headspace-thermal desorption-gas chromatography/time-of-flight mass spec", FOOD CHEMISTRY, vol. 158, 28 February 2014 (2014-02-28), pages 345-350, XP028867755, ISSN: 0308-8146, DOI: 10.1016/J.FOODCHEM.2014.02.113
- JOCHEN FISCHER ET AL: "Developement of a Candidate Reference Method for the Simultaneous Quantitation of the Boar Taint Compounds Androstenone, 3[alpha]-Androstenol, 3[beta]-Androstenol, Skatole, and Indole in Pig Fat", ANALYTICAL CHEMISTRY, vol. 83, no. 17, 29 July 2011 (2011-07-29) , pages 6785-6791, XP055606193, ISSN: 0003-2700, DOI: 10.1021/ac201465q

## Description

### Field of invention

The present invention relates to simultaneously detection of compounds related to boar taint such as the boar taint responsible compounds skatole, androstenone and indole. Such a detection method is especially important at abattoirs to detect pigs or other animals with an amount of compounds related to boar taint above a threshold value. Detected pigs are often not used for human consumption requiring heating without further processing, due to unacceptable flavour and/or odour of boar taint liberated from the meat when heated. The method is fast and makes it possible to obtain results from a sample each 10 seconds and hereby more than 360 samples per hour can be analyzed with one apparatus.

### Background of invention

Pigs and especially entire male pigs (boars) but also other animals such as goats or sheep may produce an amount of skatole, androstenone and/or indole such that the meat from these animals obtains an unacceptable flavour and/or odour of boar taint both in the non-cooked meat but especially when cooking the meat.

The number of pigs with boar taint is reduced by castrating the young male pigs, however, such castration is usually performed without sedation. A Brussels Declaration calling for a voluntary ban on the surgical castration of pigs in Europe by 2018 has been signed. Furthermore, in Denmark a national declaration to stop for castration without sedation by 2018 has been issued. As sedation is a costly and time consuming process for the farmer and no method for the farmers is available, most male piglets will in all likelihood be delivered as entire male animals to the slaughterhouse. Therefore, to avoid castration of the young male pigs it becomes more important to detect pigs with boar taint such as at the slaughterhouse.

Today, at some abattoirs, boar taint is detected by assessors who are sensitive to boar taint and who are trained to identify pigs with boar taint by sniffing to heated parts of the carcasses. Although such assessors can detect carcasses with boar taint compounds, differences exist between the persons regarding detection and in agreement upon the presence of boar taint in carcasses. The perception for a trained assessor may also vary over a working day for the individual assessor. Limits of such methods are described (L. Meinert, C. Claudi-Magnussen & S. Støier, 2013. Limits for the detection of boar taint. Fleisch Wirtschaft International 2, pp 100-103).

At other abattoirs boar taint is detected in carcasses by an automated method by detection of only skatole by a colorimetric method. However, this method only has a capacity of 200 determinations per hour (Hansen-Moller 1994, J. Chromatogr B 661, 219-230). Furthermore, this method requires large amounts of solvents.

Simultaneous detection of the boar taint responsible compounds skatole, androstenone and indole is possible (Hansen-Moller 1994, J. Chromatogr B 661, 219-230) by liquid chromatography coupled with e.g. a fluorescence detector, however with today's pig meat production at abattoirs, these methods are too time consuming for on-line measurements at an abattoir. A fast and reliable detection method is needed to make it possible to sort out the pigs with boar taint above a threshold value in due time before the carcass is entering the temperature equalization room.

Also a reference method for determination of boar taint compounds has been developed (Buttinger, G., L. Karasek, P. Verlinde & T. Wenzl, 2014. In house validation of a reference method for the determination of boar taint compounds by LC-MSMS. JRC Validated methods, reference methods and measurements, European Commission). In this document a reference method is suggested where the three marker compounds (skatole, androstenone and indole), for boar taint are quantified in pork fat by isotope dilution liquid chromatography tandem mass spectrometry (LC-MS/MS). The fat is separated from the ground pork fat tissue via melting and centrifugation. The fat is spiked with isotopically labelled standards and prepared for size exclusion chromatography (SEC). The SEC purified sample is evaporated nearly to dryness although care has to be taken not to evaporate to dryness and, after addition of an injection standard, analysed by LC-MS/MS in selected reaction monitoring mode. Analysis by gas chromatography mass spectrometry (GC-MS) is also described.

Another method that has been investigated with limited success is the use of electronic noses. These instruments are designed to absorb compounds in the headspace over a sample, on a set (typically 12 - 36) of sensors. The surface of the sensors can then change response of each sensor depending on the type of material that is absorbed. However, these instruments are highly sensitive to water vapour and are not selective enough towards specific compounds when used on complex matrices like animal fat.

EP 1 233 267 describes an invention which uses headspace sampling directly on the carcasses hanging on the slaughter line and heating a part of the carcass for desorbing the boar taint components. An ion mobility spectrometer is used for detection of produced volatiles during heating. There are two main reasons why this method is not suitable for testing on-line at abattoirs. Firstly, the ambient air in the slaughter line area is highly contaminated with moisture and organic volatile compounds which may contaminate the volatile samples. Secondly, and most important, the ion mobility spectrometer in itself is not selective. This means that the acquired mass spectra will be very complex and there will inevitably be a large number of volatile compounds interfering with the analytes of interest. Furthermore, there is no possibility of using an internal standard as a reference for the measurement. Therefore, the results will depend on the quality of the heating, the surface area being heated and the amount of volatiles that are liberated and drawn into the ion mobility spectrometer. In another embodiment of the invention a gas chromatographic column can front-end the spectrometer. However, this will reduce the speed of operation to a degree that renders the method unsuited for on-line measurements at slaughter line speeds.

Fischer et al describes "Fast and solvent-free quantitation of boar odorants in pig fat by stable isotope dilution analysis-dynamic headspace-thermal desorption-gas chromatography/time-of-flight mass spectrometry" in Food Chemistry, 2014, (pp 345-350). The sample handling is very simple, requiring no solvents or reagents. From thawed samples volatile components in fat samples are desorbed thermally into a headspace and collected on a Tenax resin that is subsequently desorbed into a CG column.

Fischer also describes "Determination of the boar taint compound skatole in meat juice by means of stable isotope dilution analysis-direct immersion solid phase microextraction-gas chromatography/mass spectrometry", Meat Science, 2012, (p. 261-265).

Using a single GC column this method can deliver a result after more than 100 minutes, which is too long for slaughterhouse needs. If this method is used for on-line detection of boar taint at a slaughterhouse with 10 seconds between the pigs, there will be a need for using 228 GC columns in parallel in order to keep up with line speeds when using the GC-data of Fischer et al., 2014.

Sorensen and Engelsen describe "Measurement of Boar Taint in Porcine Fat Using a High-Throughput Gas Chromatography-Mass Spectrometry Protocol" in Journal of Agricultural and Food Chemistry, 2014 (pp. 9420-9427). The method describes pure fat samples subjected to direct dynamic headspace sampling via thermal desorption on to a Tenax Resin and gas chromatography-MS directly.

Lohne Jack J. describes "Laser diode thermal desorption mass spectrometry for the analysis of quinolone antibiotic residues in aquacultured seafood" in Rapid Commun. Mass Spectrom., 2012 (p. 2854-2864).

Lanshoeft Christian describes "Laser diode thermal desorption atmospheric pressure chemical ionization tandem mass spectrometry applied for the ultra-fast quantitative analysis of BKM120 in human plasma" , Anal Bioanal Chem, 2014 (p. 5413-5423).

This method is designed for sorting carcasses into 3 groups of high, medium and low boar taint components. The limits of detection (LOD) are 0.62 µg/g and 0.1 µg/g for androstenone and skatole respectively. As the average content of these two components in back-fat from a normal pig population may be 0.65 µg/g and 0.09 µg/g for androstenone and skatole respectively it is clear that this method does not possess adequate selectivity and specificity for sorting carcasses. Furthermore, the method does not use internal standards but relies on producing a new calibration at regular intervals for each GC column in order to compensate for drift in column characteristics.

The developed method compromises analytical accuracy for a faster analysis thereby attempting to meet demands for speed at a normal abattoir. The method of Sorensen and Engelsen would require 36 GC columns working in parallel in order to meet the requirements of a medium size European slaughterhouse.

It is preferable that any monitoring system for detecting compounds related to boar taint is rapid, precise, reliable in operation (fail-safe) and low cost. Ideally, the method would allow simultaneous or parallel detection of both skatole, androstenone and indole and give skatole, androstenone and indole levels in absolute values rather than simply detect the presence or deliver un-precise values for sorting the carcasses especially because the boar taint threshold differs between different countries. Such a monitoring system could be used to prevent the entrance of tainted carcasses into the food chain and to allow the grading of carcasses as "premium quality". Such a method is not currently available. The present invention aims to provide a method of simultaneously detecting off-note or boar taint related compounds such as androstenone, skatole and indole that overcomes problems associated with existing methods. In particular the invention aims to provide a method of quantifying androstenone, skatole and indole that is rapid, precise and low cost. Also an abattoir test system is encompassed by the method.

### Summary of invention

The present invention is defined by method claim 1 and relates to a cheap and fast method capable of detecting boar taint animals such as boar taint carcasses within less than 1 hour, preferably within less than 30 minutes computed from the time a sample from an animal is obtained. When considered in proportion to the short analysis time the method is more accurate when compared to known method for simultaneously quantifying the boar taint compounds skatole, indole and androstenone. The limit of quantification in the described method is below 0.02 mg/kg fat for skatole and below 0.1 mg/kg fat for androstenone in pork fat. The method as described herein makes it possible to analyze a sample for 3 or more compounds within a timespan of less than 10 seconds in an apparatus such as an apparatus for LDTD-MS-MS and hereby more than 360 samples per hour can be analyzed. This is many times faster than with methods known in the art.

A boat taint threshold value is used to determine the use of animal carcass. Such threshold values may differ between countries and is dependent of the intended use of the meat and fat from the animal. In Denmark the threshold value is 0.25 mg/kg for the sum of skatole and indole in lard. A threshold value for androstenone in Denmark may become between 0.8 and 2.0 mg/kg in lard depending on for what the carcass is to be used. High skatole levels can be found not only in male pigs but also in some sows, which may also be tested by the method described herein. In many countries threshold levels have been set, above which pork is deemed unsuitable for human consumption. For skatole and androstenone these threshold levels are normally 0.2-0.3 mg/kg fat and 0.7-1.3 mg/kg fat respectively. However, the threshold levels vary between different countries.

The principle of the invention relates to a method for simultaneously quantifying the amount of compounds related to boar taint in a sample, where the method comprises the basic steps of:
a. Providing a sample;
b. Desorbing such as volatilizing the sample by heating; and
c. Quantifying one or more compounds related to boar taint by mass spectrometry.

The compounds related to boar taint may be skatole, androstenone and/or indole and the sample may comprise a biological sample, such as animal tissue e.g. back fat from a slaughtered pig.

In the method desorption such as volatilizing may be performed by heating with a laser, and the sample may be dried prior to this heating.

Quantifying one or more compounds related to boar taint by mass spectrometry is preferably performed by tandem mass spectrometry (MS-MS) or by time of flight mass spectrometry (TOF-MS).

The disclosure also relates to the use of the method described above and as described elsewhere herein for simultaneously quantifying compounds related to boar taint such as the amount of skatole, androstenone and indole in a sample.

Futhermore the disclosure relates to an abattoir test system for simultaneously quantifying compounds related to boar taint such as the amount of skatole, androstenone and indole in a sample obtained from at least one slaughtered animal, where the system comprises
- Means for obtaining tissue samples from pigs,
- Means for performing pig-id and sample-id,
- Means for extracting compounds from the obtained sample
- Means for drying the sample,
- Means for vaporizing the sample,
- Means for quantifying the amount of compounds related to boar taint such as skatole, androstenone and indole by mass spectrometry detection (MS) or by a tandem mass spectrometry detection (MS-MS) or by time of flight mass spectrometry (TOF-MS) and
- Means for comparing the quantified amount of compounds related to boar taint such as skatole, androstenone and/or indole with a threshold value for each of the compounds skatole, androstenone and indole.

In the test system the means for desorbing the sample may be performed by Laser Diode Thermal Desorption (LDTD) or other means of laser desorption and ionization (LDI).

Further details of the invention are described herein below.

### Brief description of figures

Fig. 1. Detected amount of androstenone in samples relative to detected amount of deuterated androstenone internal standard.
Fig. 2. Detected amount of skatole in samples relative to detected amount of deuterated skatole internal standard.
Fig. 3. Detected amount of indole in samples relative to detected amount of deuterated indole internal standard.
Fig. 4. Concentration of Indole in the samples of Example 2.
Fig. 5. Concentration of Skatole in the samples of Example 2.
Fig. 6. Concentration of Androstenone in the samples of Example 2.

### Detailed description of the invention

An aspect of the disclosure relates to a method for simultaneously quantifying the amount of compounds related to boar taint such as skatole, androstenone and indole in a sample, the method comprises the steps of:
a. Providing a sample;
b. Desorbing such as volatilizing the sample by heating;
c. Quantifying one or more compounds related to boar taint by mass spectrometry.

The compounds related to boar taint may be selected from the group of skatole, androstenone, indole, and other compounds/markers with a mass similar or close to the mass of androstenone. Preferably the boar taint compounds are skatole, androstenone and/or indole. Examples of compounds related to boar taint are
- androstenone as 5o-androst-16-en-3-one (cas no. 18339-16-7), 3α-androstenol (cas no. 1153-51-1) and 3β-androstenol (cas no. 7148-51-8)
- skatole as 3-methyl-1*H*-indole (cas no. 83-34-1)
- indole as 1*H*-indole (cas no. 120-72-9)

The method as described herein for detecting skatole, androstenone and/or indole may thus be used for detecting e.g.:
- skatole alone,
- androstenone alone,
- indole alone,
- skatole and androstenone,
- skatole and indole.
- androstenone and indole, or
- skatole and androstenone and indole,
and each of these possibilities e.g. together with other compounds related to boar taint. Skatole, androstenone and indole are the general terms used to describe compounds related to boar taint.

The sample may be a biological sample, such as a sample of animal tissue. If obtained from a living animal the sample may be obtained by biopsy. Preferably the sample comprises tissue from a slaughtered animal.

The sample may be obtained from any animal, preferably the sample comprises tissue from a slaughtered animal of a race known to be capable of producing or depositing any of the compounds skatole, androstenone and/or indole in the meat or fat. More preferably the sample is obtained from a living or slaughtered animal selected from the group of pigs, hogs, boars, sows, sheep, lamb, hogget, muttons, deer.

The sample may comprise any tissue from the animal, preferably the sample comprises meat and/or fat. More preferably the sample comprises back fat.

In a preferred embodiment the sample comprises pork fat. It is known that some pigs deposit skatole, androstenone and/or indole in meat and/or fat and it is important to identify these pigs and use the fat and/or meat for products where the boar taint is not a problem. Preferably the sample is obtained from a boar. Most preferably the sample is back fat obtained from a boar.

The sample may be obtained from an animal by any cutting or drilling method. A fast and easy method to obtain a biopsy is to drill a sample from an animal hereby obtaining a lard worm. In a preferred embodiment a sample is drilled from a carcass e.g. by a handheld or robotic controlled pistol capable of obtaining a sample from an animal. A lard worm may be obtained by drilling a sample e.g. from the back fat. A lard worm may be about 5 mm in diameter and 15 mm long, however it may also be smaller such as between e.g. 2 and 5 mm in diameter and between e.g. 3 and 15 mm long. From a half-carcass a sample such as a lard worm may be obtained by cutting or drilling from the cut surface and into the back fat.

The sample may be obtained by an automatic method e.g. at an abattoir. A robot arm may be controlled by a robot, such as a vision based sensor system, to cut or drill a sample from the carcass or half-carcass. A vision system, either 2D or 3D, with at least one camera may be used to obtain information used when directing the robot arm to the right position of a carcass or half-carcass to obtain a sample from the animal.

The obtained sample e.g. lard worm may be weighted and/or the volume may be determined. A sample with a suitable size may have a weight of between 0.1 and 2 gram, such as between 0.2 and 1.5 gram, e.g. between 0.3 and 1.0 gram, preferably between 0.35 and 0.8 gram, more preferably between 0.4 and 0.6 gram, most preferably about 0.5 gram. Also preferred are samples between 0.1 and 0.3 gram. A sample with a suitable volume is preferably between 0.1 and 2 cm³, such as between 0.2 and 1.5 cm³, e.g. between 0.3 and 1.0 cm³, preferably between 0.35 and 0.8 cm³, more preferably between 0.4 and 0.6 cm³, most preferably about 0.5 cm³.

The weighting and/or volume determining process may be an automatic process. Preferably the sample size obtained from an animal corresponds to the desired sample weight. Length or width of a lard worm may be determined to obtain a sample of e.g. 0.1 to 0.5 gram such as about 0.5 gram. The weight and/or volume of the sample is preferably registered.

The method as described herein may comprise
a. Providing a sample;
b. Extracting compounds from the sample;
c. Desorbing the compounds by heating; and
d. Quantifying one or more compounds related to boar taint by mass spectrometry
wherein the time from initiating the extraction process and until obtaining compounds ready to be quantified by mass spectrometry is less than 30 minutes.

Extracting compounds may be performed as described elsewhere herein. Preferably a headspace technology is not used to release the compounds of interest from the sample.

For some applications it is important to obtain a test result within a short time. This is the situation when testing animals at abattoirs where animals with boar taint responsible compounds above a specific level preferably should be identified as early as possible before the carcass enters into the chilling process or before becoming cut up in primals. For the invention described herein the time from initiating the extraction process and until obtaining compounds ready to be quantified by mass spectrometry is less than 30 minutes, such as less than 25 min, e.g. less than 20 min, such as less than 19 min, e.g. less than 18 min, such as less than 17 min.

The obtained sample is desorbed such as volatilized by heating. Any suitable heating method may be used to desorb or volatilize the compounds of interest. Preferred is microwave heating and/or heating by laser. Before desorbing the sample by heating, the sample may be subjected to further steps as described herein below.

A further step of the process is ionizing one or more compounds in the desorbed sample to produce one or more ions detectable by mass spectrometry; and quantifying one or more of the ions produced in the ionizing step by mass spectrometry wherein the quantification of the one or more ions by mass spectrometry is related to the amount of compounds related to boar taint such as skatole, androstenone and indole in the sample.

The desorption may be performed by heating with a laser, such as by laser diode thermal desorption.

Laser diode thermal desorption (LDTD) is a method for making compounds absorbed in a solid accessible for measurements by mass spectroscopy. After sample pretreatment the sample is preferably placed in a well e.g. on a micro well plate such as a LazzWell plate. The well is lined with a metallic surface or another surface of a non-adhesive material which ensures that the entire sample i.e. all liquid will be directed downwards and located at the bottom of the well. Preferably the bottom of each well is made of a material with a good thermal conductivity making it easy to evaporate compounds of the sample by heating the well bottom of the micro plate. When the sample is dry or nearly dry which may be obtained by heating or natural evaporation, the bottom of the well is irradiated with a laser diode such as in the near infrared spectrum, that evaporates or rather rapidly sublimating the sample completely. The vaporized sample can be carried by a flow of gas such as air to an atmospheric pressure ionization chamber where the compounds may be protonated, subjected to proton abstraction or simply ionized giving the molecules an electric charge so they can be accelerated in the mass spectrometer. With the LDTD technique in combination with a MS-MS detector at least 6 samples can be assessed for compounds of interest every minute. Similar speed may be obtained by heating the well bottoms with other methods such as with other means of laser desorption and ionization (LDI).

If desorbing the sample with a laser the sample is preferably dried before heating with the laser to avoid sizzling when the liquid is heated.

When using the method as described herein a sample can be analyzed for 3 or more compounds within a timespan of less than 10 seconds which is the time used in an apparatus for LDTD-MS-MS or LDI-MS-MS and hereby more than 360 samples per hour can be analyzed. When only male animals are analysed and with a speed of at least 360 animals per hour this corresponds to a capacity of at least 720 slaughtered animals per hour.

For the invention described herein the time from initiating the extraction of sample from the carcass and until obtaining the first result from a serial analysis by MS is less than 60 minutes, such as less than 55 min, e.g. less than 50 min, such as less than 45 min, e.g. less than 40 min, such as less than 35 min.

The quantification of the compounds related to boar taint may be running as serial MS-analysis such as LDTD-MS-MS or LDI-MS-MS where compounds located in and released from wells of cassettes can be analyzed. The timespan between obtaining results from two succeeding samples in a serial analysis is preferably 10 seconds or less than 10 seconds, such as 9 seconds or less than 9 seconds, e.g. 8 seconds or less than 8 seconds, such as 7 seconds or less than 7 seconds, e.g. 6 seconds or less than 6 seconds.

Below is described some possibilities for pretreating the sample before desorbing the compounds from the sample by heating.

At least one of the compounds related to boar taint such as skatole, androstenone and indole in a sample may be derivatised before desorption. Preferably skatole and indole are derivatised to increase the molecular weight of these compounds and reduce or avoid evaporation of skatole and indole during sample pretreatment such as any heating prior to desorption. Derivatisation may be performed with any known compounds suitable for derivatizing androstenone, skatole and/or indole, preferably skatole and indole are derivatised with benzyl bromide.

Ionizing the compounds and/or derivatized desorbed compounds may be performed by ionizing with any soft ionization method e.g atmospheric pressure chemical ionization (APCI). APCI is a soft ionization method which does not fragment the molecules and generally produces monocharged ions. However other soft ionization techniques capable of working at atmospheric pressure may be applied e.g. ESI (electrospray ionization) or MALDI (matrix assisted laser desorption/ionization). If using MALDI for ionization this is preferably AP-MALDI (Atmospheric pressure (AP) matrix-assisted laser desorption/ionization (MALDI)) which is an ionization technique (ion source) that operates at normal atmospheric environment. With MALDI ionization the following processes may take place. The sample is mixed with a suitable matrix material and applied to a metal plate or the sample is applied to a metal plate followed by adding a suitable matrix material. Ablation and desorption of analytes with matrix material is achieved by irradiating the sample with a pulsed laser. Before detection using MS-MS or TOF-MS, the analyte molecules are ionized by being protonated or deprotonated in the hot plume of ablated gases, and can then be accelerated into whichever mass spectrometer is used to analyse them. Ionization may be performed by e.g. ESI or APCI. Preferably ionization is performed using APCI. APCI is mainly used with polar and relatively non-polar compounds with a molecular weight of less than approximately 8000 Da such as less than 6000 Da, e.g. less than 4000 Da, such as less than 3000 Da, e.g. less than 2000 Da, such as less than 1500 Da, generally giving mono-charged ions. Ionization of the compounds is very efficient as it occurs at atmospheric pressure, and thus has a high collision frequency. Additionally, APCI considerably reduces the thermal decomposition of the compounds because of a rapid desolvation and vaporization of the droplets in the initial stages of the ionization. This combination of factors most typically results in the production of ions of the molecular species with fewer fragmentations than many other ionization methods, making it a soft ionization method.

In an embodiment, quantifying the compounds is performed by mass spectrometry (MS), such as by time-of-flight-mass spectrometry (TOF-MS). More preferably the method comprises desorbing the compounds such as by MALDI or LDTD or by other means of Laser desorption and ionization (LDI) and hereby using e.g. a MALDI-MS method or MALDI-TOF-MS method such as an AP-MALDI-MS method or AP-MALDI-TOF-MS method, respectively. The MS methods may be tandem MS methods.

In a preferred embodiment quantifying the compounds by mass spectrometry is by double mass spectrometry (MS-MS). More preferably the method comprises desorbing the compounds by laser diode thermal desorption and quantifying the compounds by mass spectrometry, hereby using a LDTD-MS-MS-method or another LDI-MS-MS method.

The method as described herein may comprises the step of
a. Providing a sample;
b. Extracting compounds related to boar taint such as skatole, androstenone and/or indole present in the sample in at least one organic solvent,
c. Optionally derivatising at least one compound present in the extracted sample,
d. Drying the extracted sample,
e. Desorbing the compounds by heating with a laser,
f. Quantifying the compounds and/or the derivatised compounds by mass spectrometry (MS) or double mass spectrometry (MS-MS) or TOF-MS.

Extracting compounds related to boar taint such as skatole, androstenone and/or indole present in the sample in at least one organic solvent may be performed with any organic solvent or combination of organic solvents e.g. acetone, methanol, n-hexane, ethyl acetate and dichloromethane. The solvent or combination of solvents may include some solvents which do not assimilate hydrogen. An example of a suitable organic solvent is acetonitrile but could be aliphatic compounds or aliphatic alcohols. Preferably a mixture comprising brine and acetonitrile is used in the extraction.

Extracting compounds related to boar taint such as skatole, androstenone and/or indole present in the sample may also be performed as a solid phase extraction (SPE) or dispersive solid phase extraction. SPE can be used for the supernatant following phase separation as described elsewhere herein, or the SPE material may be placed in direct contact with the sample e.g. a lard or meat sample obtained from an animal carcass followed by washing away the sample material leaving boar taint compounds on the SPE material and removing the boar taint material from the SPE material with organic solvent. SPE exctraction can also be used to retain undesired compounds while eluting skatole, androstenone and/or indole. Alternatively, dispersive SPE can be used for removal of contaminants e.g. by use of Quechers (Quick, Easy, Cheap, Effective, Rugged and safe) technique which involves two stages: 1) treatment of the sample with an organic solvent and salt solution and 2) treatment of the resulting extract with a dispersive solid phase extraction material. Afterwards the compounds are desorbed before quantifying the compounds related to boar taint by mass spectrometry.

Preferably internal standards are added to the sample in the extraction step. This can be performed by adding a compound very much similar to the compound(s) to be detected, but which is not naturally present in nature such as chlorinated or deuterium (= ²H-labeled) versions of the compounds to be quantified e.g by adding skatole-dn, indole-*dn* and/or androstenone-*dn*, where n is the number of ²H and for skatole n may be 1...9, for indole n may be 1...7 and for androstenone n may be 1...28. Preferably the internal standards are skatole-*d3*, indole-*d7*, androstenone-*d4* and/or androstenone-*d3.* In a preferred embodiment skatole-*d3* and androstenone-*d4* are used as internal standards. The amount of skatole-*d3* in the volume of liquid used for extraction may be 70-250 ng/mL, such as 100-225 ng/mL, e.g. 125-200 ng/mL, such as 150-190 ng/mL, e.g. 160-180 ng/mL, preferably 167 ng/mL. The amount of skatole-*d3* in the volume of liquid used for extraction may also be 25-75 ng/mL, such as 30-70 ng/mL, e.g. 35-65 ng/mL, such as 40-60 ng/mL, e.g. 45-55 ng/mL, preferably 50 ng/mL. The amount of androstenone-*d4* and/or androstenone-*d8* may be 400-600 ng/mL, such as 425-575 ng/mL, e.g. 450-550 ng/mL, such as 475-525 ng/mL, e.g. 490-510 ng/mL, preferably 500 ng/mL. Other internal standards used may be indole derivatives other than skatole that do not occur naturally in fat samples e.g. 2,3-dimethylindole, or steroids belonging to the androgen family that are not found naturally in any significant level in fat samples.

Internal standards added in known amounts to the samples make it easier to control that the obtained result for each sample is correct. A correct quantification of the content of compounds related to boar taint is important when this content is used to e.g. accept, downgrade or refuse a carcass for human consumption. Low sensitivity, too many accepted carcasses with high levels of boar taint related compounds, may result in limited or no sale to customers requiring no boar taint in the products. Low specificity, too few accepted carcasses with low levels of boar taint compounds, may result in a decrease of revenue for the abattoir as high-quality meat may be degraded to low-price products.

The amount of internal standards used in the method is preferably determined to be within the amount interval of the boar taint compound expected to be present in the samples to be analyses. If the amount of skatole and indole in a carcass sample is expected to be between 0 and 0.75 mg/kg, the amount of internal standard may be 0.25 mg/kg. If the amount of androstenone in a carcass sample is expected to be between 0 and 5 mg/kg (or even higher), the amount of internal standard may be 1 mg/kg.

In an embodiment the volume of solvent used in the method as described herein below may be reduced to 25% combined with a reduction of deuterinated androstenone and/or skatole or other internal standards also to 25% of the amounts mentioned elsewhere herein.

When using 0.5 g of sample from an animal, the boar taint compounds may be extracted by adding 1.5 mL brine and 1.5 mL organic solvent such as acetonitrile (salt assisted liquid-liquid extraction, SALLE) with internal standards (skatole-d3: 50 ng/mL and androstenone-d4: 500 ng/mL) to the sample. When a phase separation occurs the proteins and other water soluble compounds will be in the salt water phase and compounds related to boar taint such as skatole, indole and androstenone will to great extent be in the phase with the organic solvent.

Drying the extracted and/or derivatised sample may be performed by any drying method, such as by applying heat and/or directing a gas stream over the opening of the means holding the sample and hereby removing evaporating compounds. The liquid part of the sample may be evaporated nearly to dryness or to dryness. When evaporating to dryness the boar taint compounds with low molecular weights in the sample are preferably derivatised before performing the evaporation, hereby decreasing the amount of evaporated boar taint compounds and especially decreasing the amount of evaporated indole and skatole.

The method as described herein may be performed where the sample is not subjected to chromatography prior to mass spectrometry or where the sample is subjected to chromatography prior to mass spectrometry.

Preferably the compounds related to boar taint quantified by the method described herein are not obtained by liquid chromatography (LC) by the use of a LC column nor by the use of gas chromatography (GC) by the use of a GC column.

If subjecting the sample to chromatography prior to mass spectrometry this may be a liquid chromatography (LC), such as High Pressure Liquid Chromatography (HPLC) or ultra-High Pressure Liquid Chromatography (uHPLC) and may be performed before vaporizing the sample. Liquid chromatography may be performed to separate the compounds of interest from other compounds in the sample, and hereby increasing the response of or simplify the further steps in the method including the quantifying step. Subjecting the sample to chromatography prior to mass spectrometry may be performed when a rather low time to result is not important, however at an abattoir such methods may be too slow to obtain results for a certain carcass before this carcass reaches a specific location in the slaughter line or too many columns are needed to obtain the results within the required time to result. Preferably chromatography is not used at abattoirs when testing about at least 25% of the carcasses, such as about at least 50% of the carcasses being processed at the abattoir.

A method for quantifying compounds related to boar taint may include further steps and may thus comprise the steps of:
a. Obtaining a sample e.g. by taking a fat sample from the back fat of a carcass/live animal
b. Weighing and/or determining the volume of the obtained sample,
c. Homogenizing or melting the sample,
d. Adding at least one solvent spiked with internal standard(s) to the homogenized/melted sample,
e. Shaking or mixing the sample with the at least one solvent added,
f. Phase separating or centrifuging the mixed sample or awaiting phase separation of the mixed sample, obtaining a precipitate and a supernatant,
g. Subjecting the supernatant to Laser Diode Thermal Desorption (LDTD) or laser desorption and ionization (LDI) such as MALDI, High Pressure Liquid Chromatography (HPLC) or ultra-High Pressure Liquid Chromatography (uHPLC),
h. Aspirating vaporized sample into a mass spectrometry detector (MS), a tandem mass spectrometry detector (MS-MS) or a time-of-flight (TOF-MS) mass detector, and
i. Detecting or quantifying compounds related to boar taint such as skatole, androstenone and/or indole by their mass-to-charge ratio by using e.g. selective ion monitoring mode (SIM), multiple reaction monitoring (MRM) or selected reaction monitoring (SRM),
j. Quantifying the amount of compounds related to boar taint such as skatole, androstenone and/or indole in the obtained sample e.g. by relating the mass spectra of the analytes to the amount of the analytes in the original obtained sample.

When a sample which should be analyzed is obtained and weighed it may be homogenized or melted. Homogenization may be performed by any known methods such as by blending, manual cutting, and/or squeezing e.g. through a mesh. Melting the sample can be performed by gently heating the sample with microwaves or by physical contact with a warm plate. Homogenization e.g. by melting may be performed before, while and/or after adding at least one organic solvent to the sample. If homogenization by melting, this is preferably performed before adding at least one organic solvent to the sample. The sample with organic solvent may be mixed e.g. shaken to increase the movement of the boar taint compounds from the meat and/or fat tissue to the organic solvent. After the mixing, the sample may be centrifuged to separate the sample into phases, or a vial with the sample may be left standing awaiting phase separation of the shaked sample, obtaining a precipitate and a supernatant. Centrifugation may be performed with or without cooling, such as subjecting the samples to a temperature below e.g. 20°C, such as below 10 °C, e.g. below 0°C 2 °C during the centrifugation. Preferably the temperature during centrifugation is above 0°C. The phase with the organic solvent will preferably comprise the boar taint compounds, this phase may be the supernatant.

The organic solvent comprising the boar taint compounds may be subjected to Laser Diode Thermal Desorption (LDTD), or laser desorption and ionization (LDI) or High Pressure Liquid Chromatography (HPLC). Preferably the sample is subjected to LDTD or LDI.

The compounds of interest can be desorbed from the sample e.g. by the LDTD step or other means of laser desorption and ionization (LDI) followed by aspirating the liberated and ionized compounds into a mass spectrometry detector (MS) or tandem mass spectrometry detector (MS-MS) or a time-of-flight (TOF-MS) detector, to detect or quantify compounds related to boar taint such as skatole, androstenone and/or indole by their mass-to-charge ratio by using e.g. selective ion monitoring mode (SIM), multiple reaction monitoring (MRM) or selected reaction monitoring (SRM).

The mass spectra of the compounds related to boar taint such as the analytes skatole, androstenone and/or indole may be related to the amount of the analytes in the original sample by methods known in the art. Such methods may be based on the ratio between amount of quantified internal standards added to the sample and detected or quantified by MS or MS-MS. A similar ratio is expected for the amount of analytes in the sample and the amount of quantified analytes.

By including further steps or details the method for quantifying compounds related to boar taint may comprise the steps of:
a. Obtaining a sample from an animal such as from a pig in which the amount of compounds related to boar taint such as skatole, androstenone and/or indole should be determined,
b. Adding brine and organic solvent such as acetonitrile (salt assisted liquid-liquid extraction) with internal standards such as skatole-d3, indole-d7 and/or androstenone-d4 to the sample,
c. Homogenizing the sample,
d. Phase separation e.g. centrifuging the homogenized sample,
e. Selecting at least a part of the upper fraction (supernatant) with organic solvent,
f. Adding potassium hydroxide powder or another strong base such as a solution with potassium hydroxide.,
g. Optionally derivatizing skatole and/or indole with benzyl bromide,
h. Vortex,
i. Let react,
j. Adding buffer,
k. Adding organic solvents, such as non-polar and/or slightly polar organic solvents e.g Hexane and/or Ethyl acetate,
l. Vortex,
m. Phase separation, at least a part of the upper phase layer is used,
n. Deposit at least a part of the upper layer phase in a vial, in a well and/or on a plate suitable to withhold the compounds to be quantified,
o. Drying the vial, well and/or plate hereby obtaining a dried extract,
p. Subjecting the dried extract to Laser Diode Thermal Desorption (LDTD) or other means of laser desorption and ionization (LDI) where the dried extract is vaporized to obtain a vapour,
q. Ionizing e.g. with Atmospheric Pressure Chemical Ionization (APCI),
r. Aspirate the vapour into a MS-MS detector,
s. Quantifying the amount of compounds related to boar taint such as skatole, androstenone and/or indole in the vapour by tandem Mass Spectrometry detection (MS-MS) apparatus,
t. Obtaining values corresponding to the amount of compounds related to boar taint such as skatole, androstenone and/or indole in the vapour, and
u. Estimating the concentration of compounds related to boar taint such as skatole, androstenone and/or indole in the sample obtained from an animal.

The steps f to p in the list above and corresponding steps in other lists described herein may be replaced with a HPLC method such as uHPLC.

Preferred characteristics of the method described above may be any combination of:
- Obtaining a sample of a weight between 0.1 and 2 gram or of a weight and/or volume as described elsewhere herein and from an animal selected from the group of animals described elsewhere herein such as from a pig,
- Adding brine and organic solvent in a ratio between 1:2 and 2:1 e.g. 1:1 in a total amount of 2-4 mL/g sample. The organic solvent may be any polar organic solvent such as acetonitrile. The sample may be spiked by adding internal standards such as skatole-d3, indole-d7 and/or androstenone-d4 e.g. 25-75 ng/mL skatole-d3, 25-75 ng/mL indole-d7 and/or 400-600 ng/mL androstenone-d4 to the organic solvent,
- Homogenizing or shaking the sample e.g. by blending for 5-60 sec,
- Phase separation e.g. centrifuging the homogenized sample at 1,000-30,000 rpm for 5-300 sec with or without simultaneously freezing,
- Selecting at least a part of the supernatant where the selected part of the fraction may be 25 µL to 1 mL,
- Optionally adding potassium hydroxide powder, such as 10-20 mg per 100 µL supernatant, or a solution with a corresponding amount of potassium hydroxide or any other suitable strong base,
- Optionally derivatizing skatole and/or indole e.g. with benzyl bromide or derivatives of benzyl bromide such as by adding 0.25-2 µL per µL such as 0.10-2 µL per µL supernatant of a 10% (v/v) solution of benzyl bromide in acetonitrile,
- Mixing e.g. by shaking or vortexing for 2-30 sec,
- Let react for derivatizing e.g. for 0.5-15 min, such as 0.5-10 minutes, e.g. such as 1-12 min, such as 2-8 min, e.g. 3-7 min, such as 4-6 min, e.g. 0.5-2 and at a temperature of 10-80°C, e.g. 12-50 °C, such as 15-30°C, such as 20-25°C.
- Adding buffer e.g. of a pH of 7-9, such as of 7.5-8.5, in an amount of 50-400% of the total reaction volume , such as 75-350%, e.g. 100-300%, such as 150-250%, e.g. with an EDTA buffer,
- Adding Hexane:Ethyl acetate or another solvent or a solvent mixture with a low molecular weight hydrocarbon e.g. pentane or heptane, with the low molecular weight hydrocarbon such as hexane comprising above 70% of the mixture, such as 75-97%, e.g. 80-95%, such as 85-92%, and with a volume substantially equal to the volume of added buffer,
- Mixing e.g. by shaking or vortexing e.g. for 2-30 sec,
- Phase separation, e.g. by centrifuging at 1,000-30,000 g for 5-300 sec or let the phase separation occur without handling the sample for 30-600 sec, such as 60-600 sec; at least a part of the upper phase layer (with hexane:ethyl acetate) is used,
- Deposit 2-10 µL such as 2-8 µL, e.g. 2-5 µL, preferably 5 or 4 µL of the upper layer phase in a vial, in a well and/or on a plate with a wall suitable to direct liquid towards the bottom and suitable to withhold the compounds to be quantified when liquid is evaporating,
- Drying the vial, well and/or plate hereby obtaining a dried extract e.g. by heating the vial, well and/or plate in an oven, by a warm or hot stream and/or on a warm or hot plate or by letting air flow past the opening,
- Desorbing the relevant compounds from the dried extract by heating with a laser, such as be subjecting the dried extract to laser desorption e.g. Laser Diode Thermal Desorption (LDTD) or MALDI where the compounds in the dried extract are completely desorbed to the surrounding air,
- Ionizing one or more compounds e.g. indole, skatole and/or androstenone and the respective internal standards, where ionizing may be performed with Atmospheric Pressure Chemical Ionization (APCI),
- Aspirate the vapour into a detector, such as a TOF-MS detector, a MS detector or a MS-MS detector,
- Quantifying the amount of compounds related to boar taint such as skatole, androstenone and/or indole in the vapour by a Time-of-Flight (TOF) detection apparatus, a Mass Spectrometry (MS) detection apparatus or a tandem Mass Spectrometry (MS-MS) detection apparatus and
- Obtaining values corresponding to the amount of compounds related to boar taint such as skatole, androstenone and/or indole in the vapour.
- Estimating the concentration of compounds related to boar taint such as skatole, androstenone and/or indole in the sample obtained from an animal.

The vial, well or plate used for drying the sample and which is preferably suitable to direct liquid towards the bottom and suitable to withhold the compounds to be quantified when liquid is evaporating may be a LazWell plate (From Phytronix Technologies Inc) or a MALDI sample plate. Also, a strip with wells may be used.

The dried plates or strips with dry extract at the bottom of the wells may be stored until further analysis by LDTD-MS-MS or other means of laser desorption and ionization (LDI) combined with double MS.

The sample size to be analysed with the method described herein is preferably less than 2.0 g, such as less than 0.5 g, e.g. , such about 0.4 g, e.g about 0.3 g, such as about 0.2 g, e.g. about 0.1 g. The method is exemplified with a sample of about 0.5 g. For smaller samples corresponding less amounts of compounds are used in the method.

In a preferred embodiment the method for quantifying the amounts of compounds related to boar taint comprises the steps of:
a. Obtaining a sample of about 0.5 g fat from an animal such as from a pig in which the amount of compounds related to boar taint such as skatole, androstenone and/or indole should be determined,
b. Adding 1.5 mL brine and 1.5 mL organic solvent such as acetonitrile (salt assisted liquid-liquid extraction) with internal standards ( such as skatole-d3: e.g. 50 or 167 ng/mL and androstenone-d4: e.g. 500 or 1670 ng/mL) to the sample,
c. Homogenizing the sample,
d. Centrifuging the homogenized sample,
e. Selecting the upper fraction (supernatant) with acetonitrile, use e.g. 100 µl,
f. Optionally adding 10-20 mg potassium hydroxide powder,
g. Optionally derivatizing skatole and/or indole with benzyl bromide by adding 100 µl benzyl bromide (10% v/v in acetonitrile),
h. Vortex,
i. Let react for derivatizing for at least 3 minutes, such as 10 minutes at room temperature,
j. Adding 400 µL EDTA buffer (0.25 M, pH 8),
k. Adding 400 µL Hexane: Ethyl acetate (90:10),
l. Vortex,
m. Phase separation, upper phase layer is used,
n. Deposit 5 µl of upper layer phase on a micro well plate such as a LazWell well plate,
o. Drying the plate with the extract,
p. Subjecting the dried extract to laser desorption and ionization (LDI) e.g. Laser Diode Thermal Desorption (LDTD) where the dried extract is vaporized to obtain a vapour,
q. Ionizing with Atmospheric Pressure Chemical Ionization (APCI)
r. Aspirate the vapour into a MS-MS detector,
s. Quantifying the amount of compounds related to boar taint such as skatole, androstenone and/or indole in the vapour by a double Mass Spectrometry detection (MS-MS) apparatus,
t. Obtaining values corresponding to the amount of compounds related to boar taint such as skatole, androstenone and indole in the vapour and
u. Estimating the concentration of compounds related to boar taint such as skatole, androstenone and/or indole in the sample obtained from an animal.

With the method described above using a single LDTD-MS-MS system or another LDI-MS-MS e.g. MALDI-MS-MS it is possible to obtain results for individual pigs within 25 minutes after the sample is taken from the animal if the analysis is initiated just after taking the sample from the animal. It may take less than 17 minutes from receiving the sample and until the sample is ready to analyse e.g. by desorption with LDTD or another LDI method. Furthermore all sample preparation steps prior to desorption can be fully automated using laboratory robots known in the art.

The method as described herein may quantify the amount of compounds related to boar taint such as skatole, androstenone and/or indole in a MS-MS detection apparatus within less than 30 seconds, such as less than 20 seconds, e.g. less than 15 seconds, such as less the 10 seconds, e.g. less than 5 seconds calculated from the time the sample is loaded into the MS-MS detection apparatus, such as into the laser desorption unit on the MS-MS detection apparatus.

Preferably as much of the method is performed as an automatic method by automated equipment and/or robots and/or a processor. The method may be with some automatic steps or as many steps as possible may be performed as automatic steps by robots, e.g every step may be performed as automatic steps by robots. In the method the following steps may be performed as automatic steps:
a. Obtaining a sample from an animal: May be automatic if at an abattoir,
b. Entering the sample into a vial or other handling means: May be automatic,
c. Weighing the sample,
d. Adding brine and/or organic solvent with internal standards: Is preferably automatic,
e. Homogenizing the sample: Is preferably automatic,
f. Phase separation: by centrifuging, an alternative is to let the phase separation occur by itself,
g. Selecting at least a part of the upper fraction (supernatant) with organic solvent: Is preferably automatic,
h. Adding potassium hydroxide powder or solution: Is preferably automatic,
i. Adding benzyl bromide: Is preferably automatic,
j. Vortex: Is preferably automatic,
k. Let react: Is performed without handling the vial,
l. Adding buffer: Is preferably automatic,
m. Adding Hexane:Ethyl acetate: Is preferably automatic,
n. Vortex: Is preferably automatic,
o. Phase separation: May be time consuming to become automatic if including centrifuging, an alternative is to let the phase separation occur by itself,
p. Deposit at least a part of the upper layer phase in a vial, in a well and/or on a plate suitable to withhold the compounds to be quantified: Is preferably automatic,
q. Drying the vial, well and/or plate: Is preferably automatic,
r. Placing the vial, well or well plate in the vaporizing unit: Is preferably automatic,
s. Vaporizing the dried extract: Is preferably automatic,
t. Ionizing: Is preferably automatic,
u. Aspirate the vapour into a MS-MS detector: Is preferably automatic,
v. Quantifying the amount of compounds related to boar taint such as skatole, androstenone and/or indole: Is preferably automatic and
w. Estimating the concentration of compounds related to boar taint such as skatole, androstenone and/or indole in the sample obtained from an animal: Is preferably automatic.

Preferably the steps c-w are performed automatically.

When using the method with a single MS-MS apparatus it is possible to analyse more than 2,880 samples in a 16 hour work day when the apparatus is capable of testing a sample in less than 20 sec and thus more than 5,760 samples in 16 hours with an analysis result for each 10 sek or faster.

The method as described herein for simultaneously quantifying the amount of compounds related to boar taint such as skatole, androstenone and/or indole in at least one sample obtained from at least one pig may be a method performed at a laboratory away from an abattoir or at a laboratory close to an abattoir.

When the method is performed at an abattoir for identifying the animals with a concentration of compounds related to boar taint such as skatole, androstenone and/or indole in meat and/or fat above specific threshold values for one or more compounds related to boar taint, the method may comprise the steps of:
a. Marking the animals e.g. carcasses with at least one animal-id prior to or after obtaining a meat and/or fat sample from each of the animals,
b. Marking each of the obtained samples with at least one sample-id corresponding to the animal-id such that it is possible to match samples and animals based on the animal-id and the sample-id,
c. Starting with the sample, performing the steps of the method as described elsewhere herein to quantify the concentration of compounds related to boar taint such as skatole, androstenone and/or indole in the sample,
d. Comparing the obtained values of the concentration of compounds related to boar taint such as skatole, androstenone and/or indole in the sample with a threshold value for each of the compounds related to boar taint such as skatole, androstenone and/or indole,
e. Based on the comparing determining whether the sample and thus the animal has a concentration of compounds related to boar taint such as skatole, androstenone and/or indole above the threshold value,
f. For those samples having a concentration of compounds related to boar taint such as skatole, androstenone and/or indole above the threshold and based on the sample-id identify the animal with a corresponding animal-id, and
g. Removing or redirect the identified animal at the slaughter line, such as before entering the temperature chilling room after leaving the slaughter line.

An animal may be any race and/or type of animal e.g. an animal race and/or type as mentioned elsewhere herein. Preferably the animals are pigs and animal-id is pig-id.

When quantifying the concentration of compounds related to boar taint such as skatole, androstenone and/or indole in pigs at an abattoir to identify pigs with boar taint, all pigs including female and boars and hogs may be tested. In a preferred method boars and hogs of pigs are tested, but not female pigs. In another preferred embodiment for testing pigs at abattoirs only entire males i.e. boars are tested for the concentration of compounds related to boar taint such as skatole, androstenone and/or indole.

A threshold value may be determined for the concentration of at least one of the compounds related to boar taint such as skatole, androstenone and/or indole in animals when determining whether the individual animal can be used for a human food product or only for a limited number of food products and/or cannot be used for human consumption. Such a threshold value may depend on the race and/or type of animal and may also be different in different countries. A threshold value for pigs may be 250 µg/kg for indole and skatole and 1,000 µg/kg for androstenone.

An aspect of the invention relates to use of the method as described elsewhere herein for simultaneously quantifying the amount of compounds related to boar taint such as skatole, androstenone and/or indole in a sample.

In a preferred embodiment the use of the method is for identifying animals such as pigs with an amount of compounds related to boar taint such as skatole, androstenone and/or indole in the meat and/or fat above a specific threshold value for one or more of the mentioned compounds.

In the use of the method as described herein at least the following steps may be performed in a laboratory
- desorbing the compounds by heating the sample with a laser;
- quantifying the amount of one or more of the ions by mass spectrometry.

However, also further steps from taking the sample and handling the sample before desorption may be performed in a laboratory, such as ionizing one or more of the desorbed compounds from the sample to produce one or more ions detectable by mass spectrometry.

The use of the method as described herein may be for quantifying the amount of compounds related to boar taint such as skatole, androstenone and/or indole in the meat and/or fat in animals at an abattoir or in rooms connected to an abattoir.

Another aspect of the invention relates to a test system for simultaneously quantifying the amount of compounds related to boar taint such as skatole, androstenone and/or indole in a sample, such a test system may be an abattoir test system for simultaneously quantifying the amount of compounds related to boar taint such as skatole, androstenone and indole in a sample obtained from at least one slaughtered animal, and the system may comprise
a. Means for obtaining tissue samples from pigs,
b. Means for performing pig-ID and sample-ID,
c. Means for extracting compounds from said obtained sample
d. Means for drying said sample,
e. Means for vaporizing said sample,
f. Means for quantifying of the amount of compounds related to boar taint such as skatole, androstenone and indole by a Mass or tandem Mass Spectrometry detection (MS, TOF-MS or MS-MS) and
g. Means for comparing said quantifyed amount of compounds related to boar taint such as skatole, androstenone and/or indole with a threshold value for each of said compounds related to boar taint such as skatole, androstenone and indole.

The means for obtaining samples from pigs may comprise a bore pistol, such as a manual bore pistol or an automatic bore pistol handled by a robot.

The means of the test system for performing pig-id and sample-id may comprise an ID reader for reading ID of grambles and ID reader for reading ID bar codes on vials. Means for performing sample-ID may also comprise an ID reader for reading ID bar code on plates or RFID chips in plates to be entered into the Mass Spectrometer.

Means for extracting compounds from the obtained sample and preparing the sample for heating by a laser may comprise any solvents and reagents as described elsewhere herein.

The test system comprises means for desorbing compounds from the sample, where said desorption preferably is performed by Laser Diode Thermal Desorption (LDTD) or other means of laser desorption and ionization (LDI) where the ionization can be performed using APCI.

The means for detecting the amount of compounds related to boar taint such as skatole, androstenone and indole may be a double Mass Spectrometer capable of performing MS-MS.

In the test system the means for comparing the quantified amount of compounds related to boar taint such as skatole, androstenone and/or indole with a threshold value for each of said compounds related to boar taint such as skatole, androstenone and indole comprises a database and a computer and/or a processor and means for transferring results and/or information from the mass spectrometer to a dababase/processor to determine whether the amount of compounds related to boar taint such as skatole, androstenone and/or indole is below or above a predetermined threshold value and based on this determination the system may automatically determine what the animal/carcass can be used for.

The test system is preferably an automatic test system wherein at least the steps from adding solvents to the sample to detection of the amount of compounds related to boar taint such as skatole, androstenone and/or indole are performed automatically.

The test system may comprise means for releasing a message or signal when the comparing of the detected or quantified amount of compounds related to boar taint such as skatole, androstenone and/or indole with a threshold value for each of said compounds related to boar taint such as skatole, androstenone and indole indicates values above said threshold value for at least one of said compounds in an individual animal such as a pig.

The test system is preferably used for testing amount of compounds related to boar taint such as skatole, androstenone and/or indole in pig carcasses.

The test system may release a message or signal when a predetermined threshold value is reached, such a threshold value may be selected between 200-400 µg/kg for indole and skatole and 500-2,000 µg/kg for androstenone for e.g. pigs. The sample can be obtained from a slaughtered animal (a carcass) as soon as possible after the animal is dead, or as a biopsy from the back fat on a live animal. Preferably the sample is obtained from a carcass after the carcass has been cleaned on the slaughter line at the abattoir.

Another aspect of the invention relates to use of a LDTD-system or another laser desorption and ionization (LDI) system and a MS-MS system for simultaneously quantifying compounds related to boar taint such as skatole, androstenone and/or indole where a sample is pretreated as described elsewhere herein. Preferably the sample is obtained from an animal such as from a pig.

The use of a LDTD-system and MS-MS system may be with a LDTD S-960 model from Phytronix Technologies and a QTRAP 4500 or 6500 MS-MS from AB Sciex or equivalent.

The abattoir test system as described herein may perform the quantification of at least one compound related to boar taint, such as simultaneously quantifying of at least skatole, androstenone and indole with the following timespans:
- Obtaining the meat/fat-sample from a carcass and deliver this to a laboratory where the quantification is performed including transfer of the sample to a test vial such as a cassette: This time is dependent on how long time it actually takes to remove a meat/fat-sample from a carcass, locate the sample in a sample transport means, such as vials, close this sample transport means and transport the sample transport means by a sample transport system to a laboratory where the quantification can be performed. An estimate of this time could be 2 minutes for single samples, however if e.g. 16 samples are collected in a cassette the time from initiating obtaining the first sample and until the cassette reaches the laboratory including 16 samples may take e.g. 5-7.7 minutes, of which about 5 minutes may be for collecting the samples and locate these in the cassette. The samples collected in the cassette may at this stage be added liquid such as a solvent and the homogenization can be performed, hereby the sample is ready to step d) or e) in the method described in Example 1. The identification of the pig should be registered, which may be an automatic. Process. The ID of the pig can be linked to a bar code or an RFID in the cassette and to the position in the cassette. The laboratory robot and MS-system acquire the ID system making it possible to deliver the analysis results together with the pig ID of the pig from where the sample was obtained.
- It need only take less than 30 minutes from adding organic solvent to the sample in a test vial such as a cassette and until the compounds are ready to be analyzed, such as being ready to be deposited in e.g. a well with a bottom of a material with a good thermal conductivity.
- Analyzing the samples by MS and obtaining results as the amount of compounds related to boar taint in the sample may take 4 minutes. However, as samples preferably are analyzed in series there need only be less than 10 seconds between obtaining the results from two subsequent samples.
- Automatic grading of a carcass by a processor due to the content of compounds related to boar taint and forwarding result of degrading or refusal of carcasses can be done very fast.
- Removal of carcasses determined to contain compounds related to boar taint above a specific threshold level may be done automatically or manually, and is dependent on the process at the abattoir.

Some abattoirs may prefer carcasses containing compounds related to boar taint above a specific threshold level to be redirected from the line before chilling. This is possible when quantifying the compounds related to boar taint by the method described herein.

### Detailed description of the figures

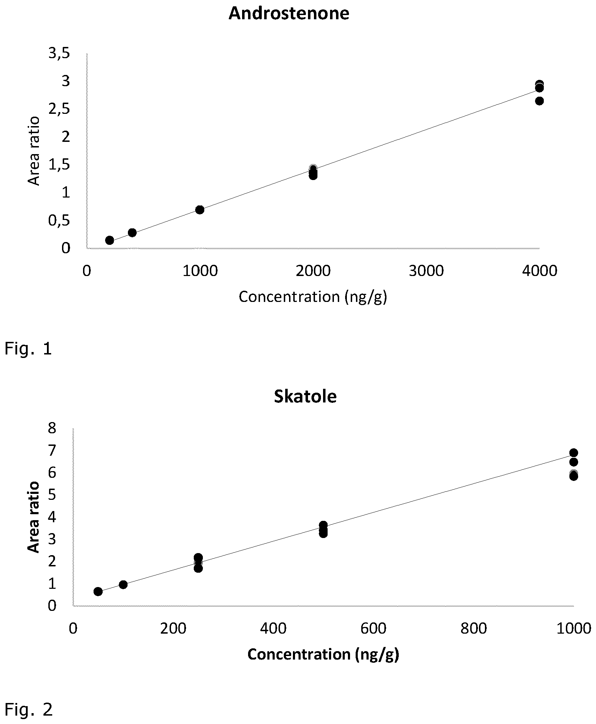

Fig. 1. Detected amount of androstenone fragment 215 Da in Example 1 in samples relative to detected amount of deuterated androstenone internal standard. Area ratio as a function of concentration ratio. Y=6.88738e⁻⁴ x + 0.00319; R² = 0.99920; Regression weighting: 1/x.

Fig. 2. Detected amount of skatole fragment - 91 Da in Example 1 in samples relative to detected amount of deuterated skatole internal standard. Area ratio as a function of concentration ratio. Y=0.00633 x + 0.32693; R² = 0.99619; Regression weighting: 1/x.

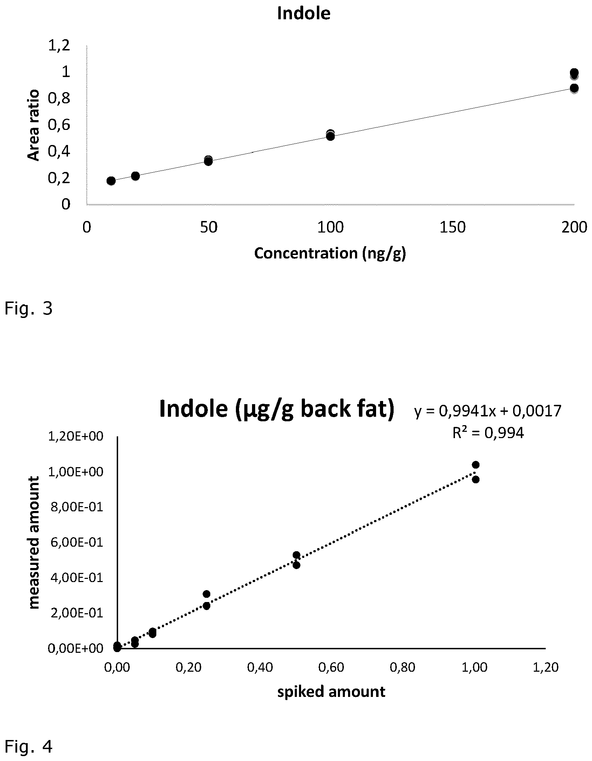

Fig. 3. Detected amount of indole fragment - 91 Da in Example 1 in samples relative to detected amount of deuterated indol internal standard. Area ratio as a function of concentration ratio. Y=0.00382 x + 0.13902; R² = 0.99188; Regression weighting: 1/x.

Fig. 4. Concentration of Indole in the samples of Example 2.

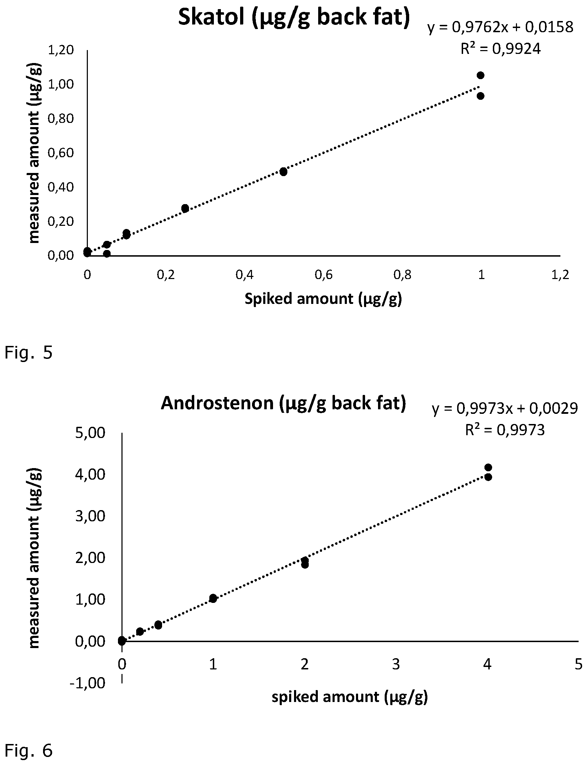

Fig. 5. Concentration of Skatole in the samples of Example 2.

Fig. 6. Concentration of Androstenone in the samples of Example 2.

### Examples

### Example 1

The developed method for quantifying compounds related to boar taint such as skatole, androstenone and/or indole has been tested using a LDTD-384 from Phytronix Technologies Inc. mounted on a MS-MS system from AB-Sciex. The method was tested on skatole, androstenone and indole and comprised the steps of:
a. Obtaining a sample of about 0.5 g fat from a pig in which the amount of skatole, androstenone and/or indole should be determined,
b. Adding 1.5 mL brine and 1.5 mL acetonitrile with internal standards (skatole-d3: 50 ng/mL and androstenone-d4: 500 ng/mL) to the sample,
c. Homogenizing the sample,
d. Centrifuging the homogenized sample,
e. Selecting 100 µl of the supernatant,
f. Adding 10-20 mg potassium hydroxide powder,
g. Derivatizing skatole and/or indole with benzyl bromide by adding 100 µl benzyl bromide (10% v/v in acetonitrile),
h. Vortex,
i. Let react for derivatizing for 3 minutes,
j. Adding 400 µL EDTA buffer (0.25 M, pH 8),
k. Adding 400 µL Hexane: Ethyl acetate (90:10),
l. Vortex,
m. Phase separation, upper phase layer is used,
n. Deposit 5 µl of upper layer phase on a LazWell plate,
o. Drying the plate with the extract,
p. Subjecting the dried extract to Laser Diode Thermal Desorption (LDTD) where the compounds are liberated to the air flow,
q. Ionizing with Atmospheric Pressure Chemical Ionization (APCI),
r. Aspirate the ionized compounds into a MS-MS detector,
s. Quantifying the liberated amount of skatole, androstenone and/or indole by a double Mass Spectrometry detection (MS-MS) apparatus,
t. Estimating or calculating the concentration of skatole, androstenone and/or indole in the sample obtained from an animal.

The dry LazWell plate is designed with a metal sheet insert allowing the liquid to be collected at the bottom of the well. Drying the plate after deposition the sample into the well was performed without handling the plate and at room temperature. In a Laser Diode Thermal Desorption (LDTD) ionization source (from Phytronix Technologies) a Laser diode heated the back-side of the well to produce a rapid thermal desorption of the dried sample. Intact desorbed molecules were carried by an air flow to an APCI region to undergo ionization. The APCI was achieved without the presence of solvent or mobile phase.

Instruments used for the analysis:
- LDTD S-960 model from Phytronix Technologies
   ∘ Ionization mode: Positive
   ∘ Carrier gas set at 3L/min air
- QTRAP 5500 from AB Sciex

### Experimental validation of the method

- Five samples of backfat from a gilt (young sow that has not farrowed) known to contain no androstenone, skatole and insignificant levels of indole were spiked with 5 levels of androstenone, skatole and indole (STD1 - STD5).
- Fat samples weighing 0.5 g from each level were subjected to SALLE extraction (brine + acetonitrile). The acetonitrile was itself spiked with the internal references skatole-d3 and androstenone-d4 to the concentrations 50 ng/ml and 500 ng/ml respectively. The skatole-d3 also served as internal standard for indole as these behave very similar in the extraction process. The process described above in the steps c-t were performed with the following system parameters:
- LDTD Gas flow: 3L/min, LDTD Laser pattern: 6 sec steady increase from 0% to 35%, 2 sec hold at 35% power level. Collision energy at MS Q3 (CE) = 25.
- MS-MS Q1 tuned in at 273 Da for androstenone, 277 Da for androstenone-d4, 222 Da for skatole, 225 Da for skatole-d3, 208 Da for indole.
- MS-MS Q3 tuned in at 215 Da for androstenone, 215 Da for androstenone-d4, 91 Da for skatole, 91 Da for skatole-d3, 91 Da for indole.
- Results for the spiked fat samples are shown in the tables below and in the figures 1-3 as the ratio between measured undeuterated components to deuterated internal standards. From these the actual concentrations can be readily calculated.

### Results:

### Androstenone

| **Standard ID** | **Nominal Conc. (ppb)** | **N** | **Mean (ppb)** | **SD** | **Precision %CV** | **Accuracy %Nominal** |
|---|---|---|---|---|---|---|
| STD1 | 200 | 4 | 204.9 | 12.8 | 6.2 | 102.5 |
| STD2 | 400 | 4 | 387.5 | 16.0 | 4.1 | 96.9 |
| STD3 | 1000 | 4 | 987.6 | 11.6 | 1.2 | 98.8 |
| STD4 | 2000 | 4 | 2056.3 | 45.8 | 2.2 | 102.8 |
| STD5 | 4000 | 4 | 3963.7 | 133.4 | 3.4 | 99.1 |

### Skatole

| **Standard ID** | **Nominal Conc. (ppb)** | **N** | **Mean (ppb)** | **SD** | **Precision %CV** | **Accuracy %Nom** |
|---|---|---|---|---|---|---|
| STD1 | 50 | 4 | 46.4 | 7.5 | 16.2 | 92.7 |
| STD2 | 100 | 4 | 100.6 | 3.1 | 3.1 | 100.6 |
| STD3 | 250 | 4 | 269.6 | 24.6 | 9.1 | 107.8 |
| STD4 | 500 | 4 | 505.2 | 23.7 | 4.7 | 101.0 |
| STD5 | 1000 | 4 | 971.1 | 61.2 | 6.3 | 97.1 |

### Indole

| **Standard ID** | **Nominal Conc. (ppb)** | **N** | **Mean (ppb)** | **SD** | **Precision %CV** | **Accuracy %Nom** |
|---|---|---|---|---|---|---|
| STD1 | 10 | 4 | 10.0 | 2.0 | 20.3 | 100.2 |
| STD2 | 20 | 4 | 19.4 | 3.3 | 17.0 | 97.2 |
| STD3 | 50 | 4 | 56.8 | 4.7 | 8.3 | 113.5 |
| STD4 | 100 | 4 | 94.7 | 7.1 | 7.5 | 94.7 |
| STD5 | 200 | 4 | 202.5 | 22.4 | 11.0 | 101.2 |

The results are also shown in figure 1-3.

### Example 2

In one series of experiments performed for testing the method the following procedure was applied.

### Calibration standards

From back fat, samples with known concentrations of androstenone, skatole and indole, provided by the reference method, 0.5 g samples were extracted. A standard addition method was used as calibration method.

### Internal standard solution

A solution was prepared containing concentrations of skatole-d3 and androstenone-d4 at 0.167 µg/mL and 1.67 µg/mL respectively, in acetonitrile.

### Standard solution

A standard stock solution was prepared containing androstenone, skatole and indole at concentrations 66.667 µg/mL, 16.667 µg/mL and 16.667 µg/mL respectively, in acetonitrile.

Back fat samples (0.5 g) were added 1.5 ml brine, 1.5 ml of the internal standard solution and the standard solution. The samples for the calibration curve were spiked as described in the table below and then subjected to the sample treatment (see below):

| Sample no. | No of vials | Spiked with | [Androst] | [Skatole] | [Indole] |
|---|---|---|---|---|---|
| 0 | 4 | 0 µL | 0 µg/g | 0 µg/g | 0 µg/g |
| 1 | 2 | 2 µL | 0.2 µg/g | 0.05 µg/g | 0.05 µg/g |
| 2 | 2 | 4 µL | 0.4 µg/g | 0.1 µg/g | 0.1 µg/g |
| 3 | 2 | 10 µL | 1.0 µg/g | 0.25 µg/g | 0.25 µg/g |
| 4 | 2 | 20 µL | 2.0 µg/g | 0.5 µg/g | 0.5 µg/g |
| 5 | 2 | 40 µL | 4.0 µg/g | 1.0 µg/g | 1.0 µg/g |

### Sample treatment:

Back fat (0.5 g) was added internal standard solution (1.5 mL) and brine (1.5 mL), followed by homogenization and centrifugation at 4000g for 5 minutes. The supernatant (100 µL) was transferred to a 1.5 mL vial containing KOH (10-20 mg). 100 µL Benzyl bromide solution (10% Bn-Br v/v in acetonitrile) was added to the vial and the mixture was vortexed for 10 seconds. After 5-15 minutes at room temperature, EDTA (400 µL, 0.25 M, pH 8) and 9:1 hexane:EtOAc (400 µL) were added. The mixture was vortexed for 10 seconds. Before MS/MS analysis 5 µL of the supernatant were transferred to the LDTD microplate and dried to dryness.

### Analysis by LDTD-MS-MS

Carrier gas flow in LDTD (3 L/min Air).

LDTD laser power output profile: 3 seconds gradient from 0 to 40% max output followed by a 2 seconds hold.

MS/MS settings for Q1 (selected ion masses) and for Q3 ion masses used for quantification:

| | Q1 mass | Q3 mass |
|---|---|---|
| Androstenone | 273.1 | 215.2 |
| Skatole derivative | 222.1 | 144.0 |
| Indole | 208.1 | 91.0 |
| Androstenone-d4 | 277.0 | 215.2 |
| Skatole-d3 | 225.0 | 147.1 |

### Results

| Indole Concentration µg/g | |
|---|---|
| Spiked | Measured |
| 0 | 1.76E-02 |
| 0.05 | 2.47E-02 |
| 0.1 | 9.57E-02 |
| 0.251 | 3.07E-01 |
| 0.503 | 5.29E-01 |
| 1.005 | 1.04E+00 |
| 0 | 4.05E-04 |
| 0 | 7.19E-03 |
| 0.05 | 4.62E-02 |
| 0.1 | 8.14E-02 |
| 0.251 | 2.40E-01 |
| 0.503 | 4.71E-01 |
| 1.005 | 9.57E-01 |
| 0 | 2.65E-03 |

| Skatole Concentration µg/g | |
|---|---|
| Spiked | Measured |
| 0 | 2.58E-02 |
| 0.05 | 6.42E-02 |
| 0.1 | 1.33E-01 |
| 0.249 | 2.73E-01 |
| 0.499 | 4.93E-01 |
| 0.998 | 9.32E-01 |
| 0 | 2.74E-02 |
| 0 | 1.40E-02 |
| 0.05 | 1.11E-02 |
| 0.1 | 1.17E-01 |
| 0.249 | 2.79E-01 |
| 0.499 | 4.86E-01 |
| 0.998 | 1.05E+00 |
| 0 | 1.38E-02 |

| Androstenone Concentration µg/g | |
|---|---|
| Spiked | Measured |
| 0 | 3.54E-02 |
| 0.2 | 2.29E-01 |
| 0.4 | 4.07E-01 |
| 1.002 | 1.01E+00 |
| 2.005 | 1.94E+00 |
| 4.01 | 3.94E+00 |
| 0 | 1.97E-02 |
| 0 | -1.15E-02 |
| 0.2 | 2.40E-01 |
| 0.4 | 3.77E-01 |
| 1.002 | 1.05E+00 |
| 2.005 | 1.83E+00 |
| 4.01 | 4.17E+00 |
| 0 | 2.05E-03 |

The results are shown as graphs in figure 4-6.

### Example 3

This example illustrates the speed of the method described herein. In this calculation it is assumed that samples are acquired at the slaughter line and placed in a cassette with room for 24 back fat samples. The table below demonstrates how the abattoir's demands for speed of operation can be accommodated with the present invention as illustrated in Example 1; one set of results is achieved every 10th second and there is a response time no longer than approximately 30 minutes.

### Sample per cassette: 24

Sample obtained from a carcass e.g. only male carcass: every 10th second.

In the table lapsed time means the amount of time (in minutes) from the time the first cassette was started being filled on the slaughter line and until the mentioned process is performed for each of 4 cassettes.

| **Slaughterline activities** | **process time (sec)** | **lapsed time (min)** |
|---|---|---|
| | | |
| Filling of cassette with samples | 240 | 4.0 |
| Cassette is weighed during filling whereby the weight of each sample is determined | | |
| Transport of cassette with samples to laboratory | 200 | 7.3 |

| **Samples in each cassette processed in parallel in a laboratory robotic workstation** | **Process time (sec)** | **Lapsed time in minutes from first cassette has started filling on the slaughter line** | | | |
|---|---|---|---|---|---|
| | | **Cass. 1** | **Cass. 2** | **Cass. 3** | **Cass. 4** |
| Loading cassettes to workstation (e.g. a scara robot) | 20 | 7.7 | 11.7 | 15.7 | 19.7 |
| Adding brine, organic solvent with internal standard | 40 | 8.3 | 12.3 | 16.3 | 20.3 |
| Homogenization | 60 | 9.3 | 13.3 | 17.3 | 21.3 |
| Centrifugation (± freezing) | 300 | 14.3 | 18.3 | 22.3 | 26.3 |
| Selecting supernatant | 20 | 14.7 | 18.7 | 22.7 | 26.7 |
| Adding derivatizing agents | 40 | 15.3 | 19.3 | 23.3 | 27.3 |
| Reaction time and vortex | 300 | 20.3 | 24.3 | 28.3 | 32.3 |
| Add EDTA and hexane | 30 | 20.8 | 24.8 | 28.8 | 32.8 |
| Vortex | 10 | 21.0 | 25.0 | 29.0 | 33.0 |
| Phase separation | 60 | 22.0 | 26.0 | 30.0 | 34.0 |
| Deposit 5µL in microtiter plate | 60 | 23.0 | 27.0 | 31.0 | 35.0 |
| Drying of samples in microtiter plate | 30 | 23.5 | 27.5 | 31.5 | 35.5 |
| Transfer plate to desorption apparatus or MS (scara robot) | 200 | 26.8 | 30.8 | 34.8 | 38.8 |
| Analysis result for the first sample in the cassette | 10 | 27.0 | 31.0 | 35.0 | 39.0 |
| Analysis result for the last sample in the cassette | 240 | 31.0 | 35.0 | 39.0 | 43.0 |

The first result may thus be obtained after 27 minutes and the 96 samples are analysed after 43 minutes with a result obtained for each 10 seconds.

## Claims

1. A method suitable for testing at an abattoir, for quantifying or simultaneously quantifying, the amounts of a compound selected from skatole and/or androstenone in a sample of animal fat tissue, said method comprising the subsequent steps of:
a. providing a sample of the animal fat tissue;
b. adding brine and an organic polar solvent, and at least one internal standard, to the sample of step a;
c. extracting the compound(s) in question from the sample of step a to obtain a precipitate and an upper fraction, said upper fraction comprising the compound(s) in question;
d. drying the separated upper phase layer of the mixture of step c to obtain a dried extract;
e. desorbing the compound(s) of the dried extract obtained in step d, by volatilizing by Laser Diode Thermal Desorption (LDTD); and
f. quantifying the desorbed and vaporized compound(s) of step e by Mass Spectrometry (MS).

2. The method of claim 1, wherein the time from initiating the extraction process (step c) and until obtaining the compound is ready to be quantified by mass spectrometry (step f) is less than 30 minutes.

3. The method of claim 1 or 2, wherein in step d, the separated upper phase layer is dried by applying heat and/or by directing a gas stream to the sample, to obtain the dried extract.

4. The method of any one of claims 1-3, wherein the Mass Spectrometry (MS) method of step f is tandem Mass Spectrometry detection (MS-MS) or time of flight mass spectrometry (TOF-MS).

5. The method of any one of claims 1-4, wherein the sample of animal fat tissue is obtained from a member selected from the group consisting of pigs, hogs, boars, sows, sheep, lambs, hoggets, muttons, and deers.

6. The method of any one of claims 1-5, wherein said sample comprises back fat.

7. The method of any one of the previous claims, wherein, in step b, the internal standard is a deuterated version of skatol and/or androstenone.

8. The method of claim 1, **characterised by** comprising the following subsequent steps executed between step c and step d:
c1. adding a strong base to the upper fraction of step c;
c2. derivatizing one or more of said compounds from the fraction of step c1 with a derivatizing agent;
c3. adding buffer to upper fraction of step c2 comprising said one or more compound; and
c4. adding a non-polar and/or slightly polar organic solvent to the upper fraction comprising one or more of the derivatized compounds of step c3, to obtain a mixture of buffer, solvent and one or more of said compounds.

9. The method of claim 8, wherein the derivatizing agent is a benzyl bromide.

10. The method of claim 8 or 9, wherein the organic polar solvent of step b is acetonitrile.

11. The method of anyone of claims 8-10, wherein the organic solvent of step e is hexane, pentane or heptane and/or ethyl acetate.

12. The method of claim anyone of claims 8-11, wherein the buffer of step d is EDTA.

## Patentansprüche

1. Ein zum Testen in einem Schlachthof geeignetes Verfahren zur Quantifizierung oder gleichzeitigen Quantifizierung der Mengen einer Verbindung, ausgewählt aus Skatol und/oder Androstenon, in einer Probe tierischen Fettgewebes, wobei das Verfahren die folgenden Schritte umfasst:
a. Bereitstellung einer Probe des tierischen Fettgewebes;
b. Zugabe von Kochsalzlösung und einem organischen polaren Lösungsmittel sowie mindestens einem internen Standard zur Probe aus Schritt a;
c. Extrahieren der fraglichen Verbindung(en) aus der Probe von Schritt a, um einen Niederschlag und eine obere Fraktion zu erhalten, wobei die obere Fraktion die fragliche Verbindung(en) umfasst;
d. Trocknen der abgetrennten oberen Phasenschicht der Mischung aus Schritt c, um einen getrockneten Extrakt zu erhalten;
e. Desorbieren der Verbindung(en) des in Schritt d erhaltenen Trockenextrakts durch Verflüchtigung durch Laserdioden-Thermodesorption (LDTD); und
f. Quantifizierung der desorbierten und verdampften Verbindung(en) von Schritt e mittels Massenspektrometrie (MS).

2. Verfahren nach Anspruch 1, wobei die Zeit vom Beginn des Extraktionsprozesses (Schritt c) bis zum Erhalt der Verbindung, die zur Quantifizierung durch Massenspektrometrie bereit ist (Schritt f), weniger als 30 Minuten beträgt.

3. Verfahren nach Anspruch 1 oder 2, wobei in Schritt d die abgetrennte obere Phasenschicht durch Anwendung von Wärme und/oder durch Richten eines Gasstroms auf die Probe getrocknet wird, um den getrockneten Extrakt zu erhalten.

4. Verfahren nach einem der Ansprüche 1-3, wobei das Massenspektrometrie-Verfahren (MS) von Schritt f eine Tandem-Massenspektrometrie-Detektion (MS-MS) oder Flugzeit-Massenspektrometrie (TOF-MS) ist.

5. Verfahren nach einem der Ansprüche 1-4, wobei die Probe tierischen Fettgewebes von einem Mitglied ausgewählt aus der Gruppe bestehend aus Schweinen, Schweinen, Wildschweinen, Sauen, Schafen, Lämmern, Schweinen, Hammeln und Hirschen gewonnen wird.

6. Verfahren nach einem der Ansprüche 1-5, wobei die Probe Rückenfett umfasst.

7. Verfahren nach einem der vorhergehenden Ansprüche, wobei in Schritt b der interne Standard eine deuterierte Version von Skatol und/oder Androstenon ist.

8. Verfahren nach Anspruch 1, **dadurch gekennzeichnet, dass** es die folgenden aufeinanderfolgenden Schritte umfasst, die zwischen Schritt c und Schritt d ausgeführt werden:
c1. Hinzufügen einer starken Base zur oberen Fraktion von Schritt c;
c2. Derivatisieren einer oder mehrerer dieser Verbindungen aus der Fraktion von Schritt c1 mit einem Derivatisierungsmittel;
c3. Hinzufügen von Puffer zur oberen Fraktion von Schritt c2, die die eine oder mehrere Verbindungen umfasst; Und
c4. Hinzufügen eines unpolaren und/oder leicht polaren organischen Lösungsmittels zu der oberen Fraktion, die eine oder mehrere der derivatisierten Verbindungen von Schritt c3 umfasst, um eine Mischung aus Puffer, Lösungsmittel und einer oder mehreren dieser Verbindungen zu erhalten.

9. Verfahren nach Anspruch 8, wobei das Derivatisierungsmittel ein Benzylbromid ist.

10. Verfahren nach Anspruch 8 oder 9, wobei das organische polare Lösungsmittel von Schritt b Acetonitril ist.

11. Verfahren nach einem der Ansprüche 8-10, wobei das organische Lösungsmittel von Schritt e Hexan, Pentan oder Heptan und/oder Ethylacetat ist.

12. Verfahren nach einem der Ansprüche 8-11, wobei der Puffer von Schritt d EDTA ist.

## Revendications

1. Procédé adapté pour tester en abattoir, pour quantifier ou quantifier simultanément, les quantités d'un composé choisi parmi le scatole et/ou l'androsténone dans un échantillon de tissu adipeux animal, ledit procédé comprenant les étapes suivantes:
a. fournir un échantillon du tissu adipeux animal;
b. ajouter de la saumure et un solvant polaire organique, et au moins un étalon interne, à l'échantillon de l'étape a;
c. extraire le ou les composés en question de l'échantillon de l'étape a pour obtenir un précipité et une fraction supérieure, ladite fraction supérieure comprenant le ou les composés en question;
d. sécher la couche de phase supérieure séparée du mélange de l'étape c pour obtenir un extrait sec;
e. désorber le ou les composés de l'extrait sec obtenu à l'étape d, par volatilisation par Laser Diode Thermal Desorption (LDTD); et
f. quantification du ou des composé(s) désorbé(s) et vaporisé(s) de l'étape e par Spectrométrie de Masse (MS).

2. Procédé selon la revendication 1, dans lequel le temps entre le début du processus d'extraction (étape c) et l'obtention du composé prêt à être quantifié par spectrométrie de masse (étape f) est inférieur à 30 minutes.

3. Procédé selon la revendication 1 ou 2, dans lequel à l'étape d, la couche de phase supérieure séparée est séchée en appliquant de la chaleur et/ou en dirigeant un courant gazeux vers l'échantillon, pour obtenir l'extrait séché.

4. Procédé selon l'une quelconque des revendications 1 à 3, dans lequel le procédé de spectrométrie de masse (MS) de l'étape f est une détection par spectrométrie de masse en tandem (MS-MS) ou une spectrométrie de masse à temps de vol (TOF-MS).

5. Procédé selon l'une quelconque des revendications 1 à 4, dans lequel l'échantillon de tissu adipeux animal est obtenu à partir d'un membre sélectionné dans le groupe consistant en porcs, porcs, sangliers, truies, moutons, agneaux, porcs, moutons et cerfs.

6. Procédé selon l'une quelconque des revendications 1 à 5, dans lequel ledit échantillon comprend de la graisse dorsale.

7. Procédé selon l'une quelconque des revendications précédentes, dans lequel, à l'étape b, l'étalon interne est une version deutérée du scatol et/ou de l'androsténone.

8. Procédé selon la revendication 1, **caractérisé en ce qu'**il comprend les étapes ultérieures suivantes exécutées entre l'étape c et l'étape d:
c1. ajouter une base forte à la fraction supérieure de l'étape c;
c2. Dérivation d'un ou plusieurs desdits composés de la fraction de l'étape c1 avec un agent de dérivation;
c3. ajouter un tampon à la fraction supérieure de l'étape c2 comprenant lesdits un ou plusieurs composés; et
c4. ajouter un solvant organique non polaire et/ou légèrement polaire à la fraction supérieure comprenant un ou plusieurs des composés dérivés de l'étape c3, pour obtenir un mélange de tampon, de solvant et d'un ou plusieurs desdits composés.

9. Procédé selon la revendication 8, dans lequel l'agent de dérivation est un bromure de benzyle.

10. Procédé selon la revendication 8 ou 9, dans lequel le solvant polaire organique de l'étape b est l'acétonitrile.

11. Procédé selon l'une quelconque des revendications 8 à 10, dans lequel le solvant organique de l'étape e est l'hexane, le pentane ou l'heptane et/ou l'acétate d'éthyle.

12. Procédé selon l'une quelconque des revendications 8 à 11, dans lequel le tampon de l'étape d est l'EDTA.
